# DEMANDE DE BREVET EUROPEEN

(11) **EP 0 555 938 A1**
(43) Date de publication de la demande: **18.08.1993**
(21) Numéro de dépôt: 93200411.2
(22) Date de dépôt: 15.02.1993
(51) Int. Cl.: A61K 31/00, A61K 31/215, A61K 31/19

(54) **Utilisation de dérivés phénoxyacétiques pour la restauration des fonctions nerveuse**

(30) Priorité: 13.02.1992 FR 9201597
(71) Demandeur: Albert ROLLAND S.A., F-69008 Lyon (FR)
(72) Inventeur: Rolland, Anne, 92380 Chilly Mazarin (FR)
(74) Mandataire: Burtin, Jean-François

(57) **Abrégé**

La présente invention se rapporte au domaine de la thérapeutique humaine ou animale.

Elle se rapporte plus particulièrement au domine de la chimie thérapeutique et vise, en particulier, les compositions pharmaceutiques renfermant, à titre de principe actif, au fins un p.chlorophénoxy acétate de formule générale I 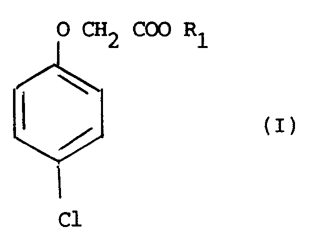
R₁ est de l'hydrogène, un cation monovalent dérivé d'une base minérale ou organise ou d'un amino acide ou bien un radical alcoyle inférieur éventuellement substitué, en association ou en mélange avec un excipient inerte, non toxique, pharmaceutiquement acceptable
Les compositions pharmaceutiques selon l'invention servent à restaurer le fonctionnement des fibres nerveuses après lésion, traumatisme ou en cas de dégénérescence.

## Description

La présente invention a pour objet une nouvelle utilisation de compositions pharmaceutiques destinées à restituer le fonctionnement des cellules nerveuses et le procédé pour leur production.

La présente invention a plus particulièrement pour objet l'utilisation de compositions pharmaceutiques dont le principe actif est un dérivé de l'acide p.chlorophénoxy acétique en association ou en mélange avec un excipient ou un véhicule inerte, en vue de la réalisation d'un médicament permettant la restauration des fonctions nerveuses.

La présente invention se rapporte, spécifiquement, à l'utilisation de compositions pharmaceutiques caractérisées en ce qu'elles renferment à titre de principe actif au moins un dérivé d'un acide p.chlorophénoxy acétique de formule générale I 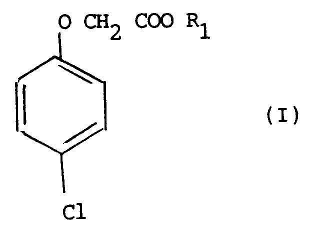
R₁ est de l'hydrogène, un cation monovalent dérivé d'une base minérale ou organique ou d'un amino acide ou bien un radical alcoyle inférieur éventuellement substitué, en association ou en mélange avec un excipient inerte, non toxique, pharmaceutiquement acceptable, en vue de la réalisation d'un médicament assurant la regénération des fonctions nerveuses.

Parmi les composés de formule générale I, on citera plus précisement les sels de métaux des acides p.chlorophénoxy acétiques comme par exemple, les sels de métaux alcalins, les sels de métaux alcalino-terreux, les sels de magnésium, les sels d'aluminium, les sels de métaux ferreux comme les sels de fer, de cobalt, de nickel ou de chrome.

On citera, plus particulièrement, parmi les sels de métaux alcalins, les sels de sodium, de potassium, de lithium ou d'ammonium, parmi les sels de métaux alcalino-terreux, les sels de calcium ou de strontium.

On citera aussi les sels de bases organiques de l'acide p.chlorophénoxy acétique comme les sels d'alcoylamine, de dialcoylamine ou de trialcoylamine, les sels de phényl alcoylamine, les sels de cycloalcoylamine, les sels de di(cycloalcoyl)amine, les sels di(cycloalcoyl) alcoylamine, les sels d'arylamine, d'arylalcoylamine ou d'hétéro arylalcoylamine, et surtout les sels de dérivés guanidiniques comme l'agmatine ou la streptidine.

On citera encore les sels d'amino acides, les sels de lysine, d'arginine ou de phényl alanine.

Parmi les esters de l'acide p.chlorophénoxy acétique de formule générale I, on relèvera les esters d'alcoyle, les esters d'alcoyle substitué par un ou plusieurs hydroxyles, libres, estérifiés ou bloqués ; par un radical amino alcoylamino ou dialcoylamino.

Parmi les esters, les composés préférés sont les esters de dialcoylamino éthyle comme l'ester de diméthylamino-éthyle, de diethylamino éthyle, de diéthylamino propyle.

Les compositions selon l'invention sont caractérisées, en outre, en ce qu'elles renferment une quantité neurologiquement active d'un composé de formule générale I comprise entre 100 et 1000 mg et de préférence de 250 à 1000 mg de composé de formule générale I par prise unitaire. La posologie journalière sera de 2 à 4 prises d'une composition pharmaceutique selon l'invention.

La quantité de principe actif de formule générale I peut varier dans de larges proportions, en fonction du poids moléculaire de la substance. La quantité est fonction également du pourcentage d'ion p.chlorophénoxy acétique présent dans la combinaison. Il est évident que la quantité neurologiquement active de composé de formule générale I sera la plus faible pour l'acide p.chlorophénoxy acétique ou son sel de lithium et la plus élevée pour un sel ou un ester d'une base ou d'un polyol complexe.

Les excipients ou les véhicules inertes utilisables sont ceux qui conviennent pour l'administration par voie parentérale, orale ou rectale. On pourra citer, à cet effet, le lactose, les amidons, la cellulose, la carboxyméthyl cellulose, le carboxyméthyl amidon, le phosphate tricalcique, le carbonate de magnésium, le stéarate de magnésium ou le talc, les solutés aqueux ou salins ou le beurre de cacao.

On pourra y adjoindre un agent liant, un agent d'adhésion, un agent facilitant la compression ou un agent facilitant l'éclatement des comprimés.

Le choix du principe actif de formule I repose sur la recherche d'un dérivé soluble si un effet rapide est recherché ou, au contraire, d'un sel ou d'un ester peu soluble dans les milieux aqueux lorsqu'on recherche un effet moins rapide mais dont la durée est plus grande. Un effet mixte peut également être recherché.

Les compositions pharmaceutiques, selon l'invention, sont obtenues selon les méthodes conventionnelles de la pharmacotechnique par mélange ou mise en contact du principe actif de formule générale I avec un excipient ou un véhicule approprié pour la voie d'administration choisie.

Les formes pharmaceutiques utilisées sont de préférence, les gélules, les capsules, les comprimés, les dragées, les comprimés effervescents, les solutés ou suspensions injectables, les sirops, les suspensions buvables, les suppositoires ou les capsules rectales.

Les compositions pharmaceutiques, selon l'invention, manifestent des propriétés pharmacologiques intéressantes et complètement inattendues. Elles exercent un effet favorable sur la regénération des nerfs après lésion traumatique ou chirurgicale.

On sait que la regénération d'une terminaison nerveuse se produit spontanément mais d'une manière remarquablement lente. De ce fait, la récupération sensitive se produit d'une manière extrêmement tardive et la récupération motrice est encore plus lente.

Les compositions pharmaceutiques selon l'invention, permettent d'accélerer ce processus et les essais sur l'animal ont mis en évidence, un effet favorable sur la regénération nerveuse après lésion, déjà significative au bout de 12 jours.

Elles peuvent donc trouver un emploi pour le traitement de neuropathies entrainant une dégénérescence des fibres nerveuses ou sensitives comme les traumatismes craniens, le syndrôme de Barré-Guillain ou certaines neuropathies d'origine diabétique.

Les exemples suivants illustrent l'invention, suis toutefois, la limiter :

### EXEMPLE I

### Préparation des sels de l'acide p.chlorophénoxy acétique de formule générale I

- Composé n° 1 :: p.chlorophénoxy acétate de lysine
On ajoute à une solution de 9,12 g de monochlorhydrate de lysine dans 1OO ml d'eau contenant 21,8 g de carbonate de sodium, une solution de 18,45 g d'acide p.chlorophénoxy acétique en solution de 100 ml d'éther, en agitant vigoureusement à 10°. L'agitation est maintenue pendant 3 heures à 20°. On dilue avec 130 ml d'eau et extrait l'éther. La solution aqueuse est acidifiée avec de l'acide chlorhydrique jusqu'à virage du papier au rouge Congo. Le précipité est lavé, séché et recristallisé 2 fois dans l'éthanol. On obtient 10,1 g (46,5%) de p.chlorophénoxy acétate de lysine, fondant à 246-248°.
- Composé n° 2 :: p.chlorophénoxy acétate d'arginine
A une solution de 17,4 g d'arginine dans 100 ml d'acide chlorhydrique normal, on ajoute à 8°, sous agitation énergique et à même vitesse, les deux solutions suivantes, sur une durée de 1 h 30 :
- 24,60 g d'acide p.chlorophénoxy acétique dans 180 ml d'éther.
- 2,2 g de carbonate de sodium dans 200 ml d'eau.
Le p.chlorophénoxy acétate d'arginine précipite dans la phase aqueuse.
Lorsque l'addition des réactifs est terminée, on maintient encore l'agitation à 8-10° pendant 3 heures. On reprend ensuite le mélange par 220 ml d'eau, acidifié avec l'acide chlorhydrique concentré, jusqu'à virage du rouge congo, et extrait à l'ether, plusieurs fois, jusqu'à absence de produit dissout dans les extraits éthérés. La phase aqueuse est alcalinisée à pH 7-8 par addition d'ammoniaque à 28 %.
Le p.chlorophénoxy acétate d'arginine reprécipite ; on l'essore après 1 h, lave à l'eau et sèche à l'étuve à 60°. Après recristallisation dans l'eau (200 ml), on obtient 20,9 g (58 %) de p.chlorophénoxy acétate d'arginine cristallisé avec une molécule d'eau, fondant à 228-230°.
- Composé n° 3 :: p.chlorophénoxy acétate de phénylalanine
8,26 g de phénylalanine en solution dans 150 ml d'eau contenant 10,6 g de carbonate de sodium, sont traités à 7° par une solution de 9,75 g d'acide p.chlorophénoxy acétique en solution dans 50 ml d'éther. Après 3 heures d'agitation à 20°, on décante l'éther, extrait la phase aqueuse à l'éther et, acidifie avec de l'acide chlorhydrique. Le précipité lavé, séché, est recristallisé dans l'alcool à 50 %. On obtient 12,6 g (75%) de p.chlorophénoxyacétate de phénylalanine, fondant avec décomposition à partir de 160°C.

### EXEMPLE DE REALISATION DE COMPOSITIONS PHARMACEUTIQUES

### EXEMPLE I

### Gélules à 0,25 g de p.chlorophénoxy acétate de sodium

| | |
|---|---|
| - p.chlorophénoxy acétate de sodium | 250 g |
| - Amidon de blé | 18 g |
| - Lactose | 110 g |
| - Talc | 2 g |
| pour 1.000 gélules | |

### EXEMPLE II

### Comprimés de 0,40 g de p.chlorophénoxy acétate de lysine

| | |
|---|---|
| - p.chlorophénoxy acétate de lysine | 410 g |
| - Cellulose microcristalline | 45 g |
| - Ethyl cellulose | 12 g |
| - Polyvinyl polypyrrolidone | 13 g |
| - Talc | 6,50 g |
| - Stéarate de Magnésium | 6,50 g |
| pour 1.000 comprimés d'un poids moyen de O,49 g | |

### EXEMPLE III

### Comprimés de 0,50 g de p.chlorophénoxy acétate de diméthylamino éthyle (sous forme de chlorhydrate)

| | |
|---|---|
| - Chlorhydrate de p.chlorophénoxy acétate de diméthylamino éthyle | 588,50 g |
| - Lactose | 125 g |
| - Amidon de blé | 28 g |
| - Carboxyméthyl cellulose (sel de sodium) | 6 g |
| - Talc | 2,50 g |
| pour 1.000 comprimés terminés à 0,75 g | |

### EXEMPLE IV

### Etude pharmacologique des composés selon l'invention

### a) 1ère étude

La vitesse de réinnervation sensitive après lésion du nerf sciatique et dégénérescence wallerienne a été étudiée chez le rat whistar mâle de 210 g ± 10 g. Le nerf sciatique droit a été lésé par congélation au moyen d'une cryode à cataracte portée à -170°C pendant 30 secondes en 3 points d'une même circonférence, située au tiers supérieur de la cuisse, à 90 ± 5 mm de la pénétration du nerf dans la région plantaire. Le nerf fémoris interne était excisé sur plusieurs cm. La récupération sensitive a été appréciée en mesurant la latence du potentiel évoqué somesthésique recueilli au niveau de l'aire primaire contralatérale et homolatérale, au moyen d'électrodes métalliques extra-durémériennes scéllées et laissées à demeure. La réponse était évoquée en stimulant le coussinet le plus interne de la sole plantaire droite au moyen d'un choc électrique de 5 mA délivré à courant constant pendant 2 ms toutes les 620 ms. Les réponses étaient moyennées de 500 à 2.000 fois et leurs latences mesurées avec une précision de ± 0,5 ms.

La latence de la réponse positive (P1) la plus précoce et à seuil le plus bas, a été mesurée et comparée chez trois groupes d'animaux aux jours J +25, +29, +33, +38, +43. Le groupe de témoins (n=11) a reçu par gavage 6 jours sur 7, 0,5 g/kg de gomme arabique. Le groupe traité a reçu, aux mêmes dates et par la même voie (n=5), une dose de parachlorophénoxy acétate de sodium, de 0,271 g/kg. Les courbes de régression ont été calculée par la méthode des moindres carrés et les moyennes ont été comparées par couple à l'aide du test "t" de Student.

Les résultats obtenus indiquent une accélération de la vitesse de réapparition des réponses sensitives P1 de 2,5 jours en moyenne chez les animaux traités par les sels de l'acide parachlorophénoxy acétique.

Les moyennes sont significativement différentes pour chaque point de la courbe. Entre le lot des animaux traités et celui des animaux témoins, la comparaison des moyennes par couple donne une différence significative au niveau P<O,O1 entre les témoins d'une part, et le groupe traité d'autre part. Enfin, il n'existe pas de différence significative entre les animaux traités par les divers sels de l'acide p.chlorophénoxy acétique.

Le traitement par les sels de l'acide p.chlorophénoxy acétique fait apparaitre précocement, dès le jour J +45, une réponse négative N1 à seuil plus élevé que P1 qui ne peut être observée chez les animaux témoins avant le 50ème jour après la lésion.

Les résultats obtenus conduisent à la conclusion que les sels de l'acide p.chlorophénoxy acétique ont une action puissante sur la regénération des fibres nerveuses des mammifères, ainsi qu'il ressort de la courbe I, ci-jointe.

Comparativement, l'isaxonine administrée à la dose de 150 mg/kg a un autre lot de rats, n'a entrainé aucune modification sensible dans la regénération des fibres nerveuses (Fig. 2).

### b) 2ème étude

### Etude de la regénération du nerf sciatique après exposition au froid

L'étude est menée sur des lots de rats Charles RIVER (100-110 g), anesthésiés au Mébubarbital (30 mg/kg I.P).

Le nerf sciatique droit est isolé et on en pratique la congélation sur une longueur de 3 mm, au niveau du tiers supérieur de la cuisse. Cette congélation est réalisée par applications successives, pendant 30 secondes chacune, de petites masses métalliques adaptées sur un champ d'artère et plongées, préalablement, dans l'azote liquide.

Le traitement par les produits à étudier est pratique à partir du premier jour après l'opération (J₁), jusqu'à J₁₂.

La regénération nerveuse éventuelle est recherchée par la mesure de la perception douloureuse au niveau plantaire des deux pattes postérieures des animaux, en utilisant l'appareil de Randal et Sellito qui permet l'évaluation de la sensibilité locale par application d'un poids progressivement augmenté en fonction du temps.

Les résultats sont exprimés à partir des mesures réalisées sur chaque patte, 4 heures après le dernier traitement. La valeur de la regénération de la sensibilité, donc de la regénération nerveuse, est représentée par la différence de réponse au test de la patte au nerf lésé par rapport à la patte contralatérale intacte.

L'analyse statistique est faite sur les deux séries de valeurs par analyse de Kruskall-Wallis et test U de Mann-Whitney.

Les produits selon l'invention, par exemple le Méclofénoxate, sont administrés par voie orale, en solution dans l'eau, de telle façon que le volume de solution administrée soit égal à 1 ml/100 g/rat.

### RESULTATS

Au sixième jour après l'opération, on note une tendance à une meilleure réactivité chez les animaux ayant reçu le Méclofénoxate, 500 mg/kg P.U/jour.

Au douzième jour, l'amélioration est significative ainsi qu'il ressort des résultats figurés sur la planche n° 3 ci-jointe.

On peut donc conclure que le traitement quotidien des rats permet une regénération plus rapide des nerfs lésés par congélation au temps O.

## Revendications

L'invention a pour objet :

1. Utilisation d'une composition pharmaceutique en vue de l'obtention d'un médicament destiné à restituer le fonctionnement de la cellule nerveuse caractérisée en ce qu'elle contient à titre de principe actif une quantité neurologiquement-active d'au moins un dérivé de l'acide p.chlorophénoxy acétique de formule générale I 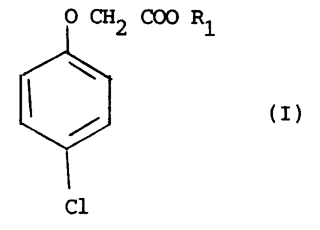 dans laquelle R₁ est de l'hydrogène, un cation monovalent dérivé d'une base minérale ou organique ou d'un amino acide ou bien un radical alcoyle inférieur éventuellement substitué, en association ou en mélange avec un excipient inerte, non toxique, pharmaceutiquement acceptable

2. Utilisation selon la revendiation 1° d'une composition pharmaceutique dans laquelle l'excipient ou le véhicule du médicament est un de ceux qui conviennent pour l'administration parentérale, orale ou rectale.

3. Utilisation selon l'une des revendications 1° ou 2° dans laquelle, la quantité neurologiquement active de composé de formule générale I varie de 100 à 1000 mg par prise unitaire.

4. Utilisation selon l'une des revendications 1° à 3° dans laquelle, la quantité neurologiquement active de composé de formule générale I varie de 250 à 1.000 mg par prise unitaire.

5. Utilisation selon l'une des revendications 1° à 4° dans laquelle, le principe actif du médicament est l'acide p.chlorophénoxy acétique.

6. Utilisation selon l'une des revendications 1° à 4° dans laquelle, le principe actif du médicament est le p.chlorophénoxy acetate de sodium.

7. Utilisation selon l'une des revendications 1° à 4° dans laquelle, le principe actif du médicament est le p.chlorophénoxy acétate de lysine.

8. Utilisation selon l'une des revendications 1° à 4° dans laquelle, le principe actif du médicament est le p.chlorophénoxy acétate de diméthylamino éthyle.

9. Utilisation selon l'un des revendications 1° à 4° dans laquelle, le principe actif du médicament est le p.chlorophénoxy acétate d'arginine.

10. Procédé de préparation des compositions pharmaceutiques qui consiste en ce qu'on mélange ou on ajoute à un composé de formule générale I, un excipient ou un véhicule inerte non toxique pharmaceutiquement-acceptable, selon les méthodes habituelles de la pharmacotechnie en vue de la réalisation du médicament selon l'une des revendications 1 à 9°.
